# EUROPÄISCHE PATENTSCHRIFT

(11) **EP 0 560 138 B1**
(45) Veröffentlichungstag und Bekanntmachung des Hinweises auf die Patenterteilung: **03.04.1996**
(21) Anmeldenummer: 93102968.0
(22) Anmeldetag: 25.02.1993
(51) Int. Cl.: A61K 9/127

(54) **Liposomale Wirkstoff-Formulierungen und Verfahren zu ihrer Herstellung**
Drug formulations by liposomes and the process for their manufacture
Formulations liposomales de produits pharmaceutiques et le procédé pour leur production

(30) Priorität: 10.03.1992 DE 4207481
(43) Veröffentlichungstag der Anmeldung: 15.09.1993
(73) Patentinhaber: BAYER AG, D-51368 Leverkusen (DE)
(72) Erfinder: Kurka, Peter, Dr., W-4010 Hilden (DE); Serno, Peter, Dr., W-5060 Bergisch Gladbach (DE); Rupp, Roland, Dr., W-5653 Leichlingen (DE); Klinksiek, Bernd, Dipl.-Ing., W-5060 Bergisch Gladbach 1 (DE); Dee, Karl-Hans, Dr., W-5000 Köln 80 (DE); Friedel, Horst-Dieter, Dr., W-5090 Leverkusen 1 (DE); Herboth, Matthias, Dr., W-5090 Leverkusen 1 (DE)

(56) Entgegenhaltungen:
- EP-A- 0 200 068
- EP-A- 0 461 333
- DE-A- 3 339 861

## Beschreibung

Die Erfindung betrifft eine parenteral applizierbare, liposomale Dihydropyridin-Formulierung sowie ein Verfahren zu ihrer Herstellung.

Dihydropyridine sind im allgemeinen schlecht wasserlöslich. Für parenterale Applikationen ist man daher auf den Einsatz mit Wasser mischbarer, verträglicher organischer Lösungsmittel angewiesen oder auf den Einsatz partikulärer Injektions- oder Infusionsformulierungen. Zu ihnen zählen parenterale Nanoemulsionen, Nanosuspensionen (Hydrosole) und Liposomen.

Organische Lösungsmittelsysteme als Träger für Dihydropyridine weisen eine Reihe von Nachteilen auf: wegen zu geringer Verträglichkeit in den peripheren Gefäßen müssen sie entweder über einen Zentralkatheter oder verdünnt mit einer gleichzeitig applizierten Koinfusion verabreicht werden. Dabei kann es auf Grund der Löslichkeitsverhältnisse zu Komplikationen infolge Übersättigungen kommen. Weiterhin müßten in Abhängigkeit vom Dihydropyridin-Typ erhebliche Mengen an organischen Lösungsmitteln und gegebenenfalls weitere Hilfsstoffe zugefürt werden. Insbesondere bei länger anhaltenden, intravenösen Therapien würde dies zu einer nicht mehr tolerierbaren Menge von unerwünschtem Lösungsmittel führen.

Es ist bekannt, daß schwer lösliche Wirkstoffe in liposomaler Darreichungsform parenteral appliziert werden können (Liposome Technology, Vol I-III, ed. Gregoriadis). Es ist ferner bekannt, daß Liposomen-Dispersionen durch verschiedene Techniken hergestellt werden können. Als Beispiele seien genannt: Hochdruckhomogenisation (EP-A-0 101 007), Ultrabeschallung, Dialyse, Extrusion, Microfluidisation und Verdünnungsverfahren. Bei den bekannten Herstelltechniken sind auch Verfahren ohne Einsatz organischer Lösungsmittel beschrieben, die jedoch nur unter bestimmten Bedingungen funktionieren und/oder einen hohen technischen Aufwand erfordern.

Insbesondere für Nimodipin ist z.B. in DE 35 15 335 beschrieben, wie dieses besonders schwer lösliche Dihydropyridin unter Einsatz organischer Lösungsmittel liposomal formuliert werden kann.

Es wurde überraschend gefunden, daß bei schwer löslichen Wirkstoffen und selbst bei der extrem geringen Löslichkeit von Nimodipin der Einsatz organischer Lösungsmittel bei der Herstellung von Liposomen vermieden werden kann. Die erfindungsgemäßen Liposomen werden nach folgendem Verfahren hergestellt: Cryoprotector, pH-Stabilisator, Antioxidans, Phospholipid und Wirkstoff werden ohne Einsatz organischer Lösungsmittel in einer mit Stickstoff begasten Wasservorlage vorhomogenisiert. Anschließend wird die Vordispersion bei erhöhter Temperatur so lange hochdruckhomogenisiert, bis die gewünschte Liposomengröße erreicht ist.

Es war nicht vorhersehbar, daß selbst mit einem so schwer löslichen Dihydropyridin wie Nimodipin stabile Liposomen gebildet werden können, ohne daß der Wirkstoff rekristallisiert.

Es ist bekannt, daß Zusätze von Polyalkoholen (Offenlegungsschrift DE 35 15 335 A1) die Rekristallisation von Wirkstoffen wie Dihydropyridinen in Liposomen-Dispersionen verhindern können.

Es wurde überraschend gefunden, daß sich Wirkstoffe, insbesondere Dihydropyridine, bei Einhalten von Wirkstoff:Phospholipidvehältnissen von 1:20 bis 1:60 ohne Anwesenheit organischer Lösungsmittel und ohne den aus medizinischer Sicht nachteiligen Zusatz von Polyalkoholen zur Rekristallisationshemmung formulieren lassen. Bei den genannten Wirkstoff:Phospholipidverhältnissen rekristallisiert der Wirkstoff weder während der Herstellung in der Dispersion noch während der Lagerung im Lyophilisat aus. Mit dem erfindungsgemäßen Wirkstoff:Phospholipidverhältnis erreicht man also auch ohne den Zusatz von Polyalkoholen eine lagerstabile Liposomenformulierung. Insbesondere war nicht vorhersehbar, daß der Wirkstoff auch dann nicht rekristallisiert, wenn nach Lagerung von 2 Jahren bei 6°C die Liposomen Lyophilisate rekonstituiert werden.

Die Erfindung betrifft parenteral applizierbare stabile Arzneizubereitungen, frei von organischen Lösungsmitteln auf Basis von Liposomen mit Phospholipidmembran mit einem Wirkstoff:Phospholipidverhältnis zwischen 1:20 und 1:100 und einem mittleren Liposomen-Durchmesser zwischen 40 und 200 nm, die nach Rekonstitution einen pH-Wert zwischen 4,5 und 6,5 aufweisen.

Besonders bevorzugt sind Arzneizubereitungen, deren Liposomenmembran zwischen 20 und 60 Gew.-Teilen Phospholipid enthält und 1 Teil Wirkstoff enthält, wobei als bevorzugte Wirkstoffe Dihydropyridine der Formel (I) 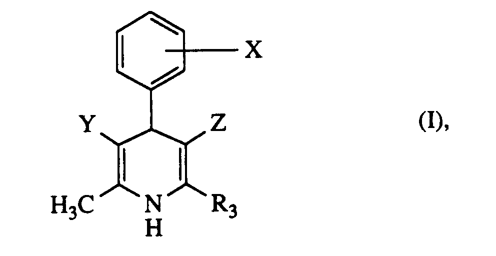
in der
- Y: die Gruppe NO₂, CN oder -COOR₁ bedeutet, wobei
- R₁: für C₁-C₄-Alkyl, gegebenenfalls substituiert durch C₁-C₃-Alkoxy, steht,
- Z: für die Gruppe CO-NH-Cycloalkyl (3-7 C-Atome) oder -COOR₂ steht, wobei
- R₂: C₁-C₁₀-Alkyl, gegebenenfalls substituiert durch C₁-C₃-Alkoxy, Trifluormethyl, N-Methyl-N-benzylamino oder Benzyl bedeutet,
- R₃: für C₁-C₄-Alkyl, Cyano oder Hydroxyalkyl (1-4 C-Atome) steht und
- X: für 1 oder 2 gleiche oder verschiedene Substituenten aus der Gruppe Nitro, Halogen, C₁-C₄-Alkyl, C₁-C₄-Alkoxy, Benzoyl, ein 2,3-Ringglied bestehend aus =N-O-N=, 2-Cycloalkylmethylthio, 2-(4-Methylbenzyloxy) oder Trifluormethyl steht,
eingesetzt werden.

Die Erfindung betrifft auch Verfahren zur Herstellung der erfindungsgemäßen Zubereitungen, dadurch gekennzeichnet, daß man 1 Gew.-Teil des Wirkstoffs und 20-60 Gew.-Teile Phospholipid zusammen mit einem Antioxidans, einem Cryoprotector und einem pH-Stabilisator bei Temperaturen zwischen ca. 10° und 80°C, vorzugsweise 20 bis 80°C, und einem pH-Wert zwischen 5 und 7,5, vorzugsweise 6 und 7, in Stickstoff begastem Wasser mit einem Rührwerk ausreichend lange vordispergiert und die Liposomen dann in einem abriebfrei arbeitenden Hochdruckdüsenhomogenisator bei Temperaturen zwischen 20°C und 80°C und einem Druck zwischen 400 und 1 000 bar auf eine mittlere Größe zwischen 40 und 200 nm homogenisiert und die so erhaltene Dispersion gegebenenfalls anschließend in geeignete vials abfüllt und schnell auf Temperaturen zwischen -50°C und - 70°C einfriert. Besonders vorteilhaft ist es, als Cryoprotector Glucose einzusetzen und ein Verhältnis zwischen Cryoprotector und Phospholipid zwischen 1:1 und 4:1 zu wählen. Gegebenenfalls können die so hergestellten Liposomen nach der Homogenisation noch entkeimt werden, z.B. durch Filtration über einen 0,2 µm-Filter.

Zur Erzielung kurzer Trocknungszeiten kann es vorteilhaft sein, die shell freeze oder die spin freeze Technik einzusetzen.

Als geeignete Rekonstitutionsmedien sei isotone Glucoselösung (5 %ig) oder Aqua pro injectione genannt. Zur Verbesserung des Flockungsverhaltens nach Rekonstitution ist es gegebenenfalls empfehlenswert, daß die Liposomenmembran bis zu 10 Gew.-% eines Ladungsinducers enthält. Bevorzugt seien genannt DMPG-Na (Dimynstoyl-Phosphatidylglycerol-Natriumsalz), DPPG-Na (Dipalmitoyl-Phosphatidylglycerol-Natriumsalz) oder DSPG-Na (Distearoyl-Phosphatidylglycerol-Natriumsalz).

Die erfindungsgemäßen Arzneizubereitungen zeichnen sich durch hervorragende Temperatur- und Lagerstabilität aus. Sie können z.B. bei Temperaturen unter 30°C mindestens zwei Jahre gelagert werden, ohne daß nach Rekonstitution Qualitätseinbußen auftreten. Die rekonstituierte Dispersionen sind bis zu 24 h stabil und zeigen keine Ausflockungs- oder Aggregationserscheinungen des Phospholipids.

Die erfindungsgemäßen Liposomen eignen sich generell für lipophile Wirkstoffe, insbesondere für Dihydropyridine wie Nifedipin, Nisoldipin, Nimodipin, Nitrendipin, Felodipin oder Amlodipin. Insbesondere eignet sich die Technik auch für sehr schwer lösliche Dihydropyridine wie Nimodipin.

Die Herstellung der erfindungsgemäßen Liposomen umfaßt mehrere Teilschritte. Zunächst wird nach Einwaage der Einsatzstoffe ein Vorhomogenisat mit üblichen Rotor-Stator-Rührwerken hergestellt. Dabei enthalten die Vordispersionen das Phospholipid und Nimodipin im genannten Verhältnis zwischen 20:1 und 40:1. Die Vordispersionen enthalten weiterhin bekannte Cryoprotectoren wie Saccharose, Glucose oder Fructose, Antioxidantien wie Ascorbinsäure und pH-Stabilisatoren wie Arginin.

Als Phospholipide zum Aufbau der Liposomen für parenterale Applikationen kommen alle Phospholipide mit der dafür üblichen Reinheit in Betracht Die Phospholipide können dabei sowohl ungesättigt als auch zur Erzielung anderer pharmakokinetischer Profile gesättigt sein. Soja- oder Ei-Phospholipide sind als natürliche Phospholipide ebenso geeignet wie synthetische Phospholipide. Zur Erzielung besonders translucenter Liposomen-Dispersionen und zur Verbesserung des Flockungsverhaltens nach Rekonstitution ist der Zusatz von bis zu 10 % Ladungsinducern wie Na-DMPG, Na-DPPG oder Na-DSPG vorteilhaft.

Das Vorhomogenisat wird vor Hochdruckhomogenisation durch 5 µm-Filter filtriert und anschließend in einem üblichen Hochdruckdüsenhomogenisator bei Temperaturen zwischen 20 und 80°C vorzugsweise zwischen 40 und 80°C unter Inertgas wie Stickstoffschutz auf die gewünschte Liposomengröße zwischen 40 und 50 nm homogenisiert.

Die homogenisierte Liposomen-Dispersion wird zum Zweck der Keimreduzierung über ein übliches 0,2 µm-Filter filtriert und anschließend lyophilisiert. Aus Gründen einer optimalen Cryoprotector Wirkung sollte das Phospholipid:Cryoprotector-Verhältnis zwischen 1:1 und 1:4, vorzugsweise zwischen 1:1,8 und 1:4 liegen.

Zum Zweck der Lyophilisation wird die Dispersion in vials oder Infusionsflaschen abgefüllt, die Flaschen in den Gefriertrockner gestellt und das Produkt dort mit Hilfe der auf -50°C vorgekühlten Stellflächen eingefroren. Zur Erzielung besonders kurzer Trocknungszeiten ist es vorteilhaft, die Flaschen oder vials rotierend nach dem Prinzip des spin oder shell freezing einzufrieren.

Die erfindungsgemäß hergestellten Liposomen sind auch nach Homogenisation zwischen ca. 40 und 50 nm groß und weisen eine enge Verteilung um diesen Mittelwert auf.

Die erfindungsgemäß hergestellten Liposomen Lyophilisate können vor Applikation mit den gängigen, parenteralen Lösungen rekonstituiert werden. Vorzugsweise erfolgt die Rekonstitution mit 5 %iger Glucoselösung oder Aqua pro injectione. Die Lyophilisate lassen sich innerhalb einer Minute unter wiederholtem Schütteln rekonstituieren.

Die erfindungsgemäß rekonstituierten Liposomen-Dispersionen werden vorzugsweise mit üblichen Infusionspumpsystemen wie Perfusor®, Imed® oder Ivac® mit der gewünschten Geschwindigkeit appliziert. Aus Sicherheitsgründen wird die rekonstituierte Liposomen-Dispersion vor Applikation durch einen handelsüblichen Filter in-line filtriert.

### Ausführungsbeispiele

### Beispiel 1

800 g Wasser für Injektionszwecke werden 10 min mit Stickstoff begast. Anschließend werden in dieser Wassermenge 1 g Ascorbinsäure, 150 g Glucose und 1 g L-Arginin gelöst. In diesem Medium werden 1,667 g Nimodipin und 83,35 g hochreines Ei-Phospholipid (z.B. Lipoid E PC) dispergiert und mit stickstoffbegastem Wasser auf 1055,5 g aufgefüllt (Wirkstoff:Phospholipid-Verhältnis = 1:50).

Diese Dispersion wird 30 min bei 75°C und unter Stickstoffschutz mit einem schnellaufenden Rührwerk (z.B. Ultra-Turrax) vorhomogenisiert Die Vordispersion wird durch einen 5 µm-Filter filtriert.

Anschließend wird die Vordispersion bei 75°C, 800 bar und Stickstoffschutz in einem abriebfrei arbeitenden Hochdruckdüsenhomogenisator eine Stunde homogenisiert. Nach dem Abkühlen auf Raumtemperatur erfolgt die Entkeimungsfiltration (0,2 µm). Der Gehalt beträgt 100 % des Sollgehalts.

Die Dispersion wird zu 15,3 ml in 250- oder 50 ml-Flaschen abgefüllt und mit Hilfe des spin- oder shell freezing bei -65°C (Trockeneis-Ethanol-Kühlmischung) zu einem dünnen Produktkuchen eingefroren.

Das eingefrorene Produkt wird auf die auf -50°C vorgekühlten Stellflächen eines Lyophilisators gebracht und bei -30°C und 0,05 mbar 14 h lang getrocknet. Die Nachtrocknung dauert 7 h bei 30°C und 0,001 mbar.

Das Lyophilisat wird mit isotoner Glucoselösung ad 250 ml oder mit Aqua p.i. ad 50 ml rekonstituiert Der Gehalt beträgt 100 % des Sollgehalts.

| | Liposomeneigenschaften: | |
|---|---|---|
| | vor Lyophilisation | nach Rekonstitution |
| mittlere Größe | 45 nm | 50 nm |
| Dispersitätsindex K2 | 0,350 | 0,500 |
| Trübung | 170 Einheiten | 40 Einheiten |
| Osmolarität | 1,2 Osmol | 360 mOsmol |
| pH | 6,5 | 4,5 |
| Partikellast (HIAC) nach in-line Filtration | | <10 Partikel (>2 µm/ml) |

### Beispiel 2

800 g Wasser für Injektionszwecke werden 10 min mit Stickstoff begast. Anschließend werden in dieser Wassermenge 1 g Ascorbinsäure, 100 g Glucose und 1 g L-Arginin gelöst. In diesem Medium werden 1,667 g Nimodipin und 50,00 g hochreines Soja-Phospholipid (z.B. Phospholipon 90) dispergiert und mit stickstoffbegastem Wasser auf 1055,5 g aufgefüllt (Wirkstoff:Phospholipid-Verhältnis =1:30).

Diese Dispersion wird 30 min bei 75°C und unter Stickstoffschutz mit einem schnellaufenden Rührwerk (z.B. Ultra-Turrax) vorhomogenisiert. Die Vordispersion wird durch einen 5 µm-Filter filtriert.

Anschließend wird die Vordispersion bei 75°C, 800 bar und Stickstoffschutz in einem abriebfrei arbeitenden Hochdruckdüsenhomogenisator eine Stunde homogenisiert. Nach dem Abkühlen auf Raumtemperatur erfolgt die Entkeimungsfiltration (0,2 µm). Der Gehalt beträgt 100 % des Sollgehalts.

Im Gegensatz zu Beispiel 1 wird nach Abfüllen in entsprechende Flaschen ohne Einsatz des spin- oder shell freezing die Dispersion bei -65°C eingefroren.

Das Produkt wird bei +30°C und 0,05 mbar 7 h getrocknet (Haupttrocknung) und bei +30°C und 0,001 mbar nachgetrocknet. Die Liposomeneigenschaften aus dem Beispiel 1 bleiben erhalten.

### Beispiel 3

800 g Wasser für Injektionszwecke werden 10 min mit Stickstoff begast. Anschließend werden in dieser Wassermenge 1 g Ascorbinsäure, 100 g Glucose und 1 g L-Arginin gelöst. In diesem Medium werden 1,667 g Nimodipin, 45 g hochreines, gesättigtes Phospholipid (z.B. Epikuron 200 SH) und 5 g DMPG-Na dispergiert und mit stickstoffbegastem Wasser auf 1055,5 g aufgefüllt (Wirkstoff:Phospholipid-Verhältnis = 1:30).

Diese Dispersion wird 30 min bei 75°C und unter Stickstoffschutz mit einem schnellaufenden Rührwerk (z.B. Ultra-Turrax) vorhomogenisiert. Die Vordispersion wird durch einen 5 µm-Filter filtriert.

Anschließend wird die Vordispersion bei 75°C, 800 bar und Stickstoffschutz in einem abriebfrei arbeitenden Hochdruckdüsenhomogenisator eine Stunde homogenisiert. Nach dem Abkühlen auf Raumtemperatur erfolgt die Entkeimungsfiltration (0,2 µm). Der Gehalt beträgt 100 % des Sollgehalts.

Die Dispersion wird zu 15,3 ml in 250 ml- oder 50 ml-Flaschen abgefüllt und mit Hilfe des spin- oder shell freezing bei -65°C (Trockeneis-Ethanol-Kühlmischung) zu einem dünnen Produktkuchen eingefroren.

Das eingefrorene Produkt wird auf die auf -50°C vorgekühlten Stellflächen eines Lyophilisators gebracht und bei -30°C und 0,05 mbar 14 h lang getrocknet. Die Nachtrocknung dauert 7 h bei 30°C und 0,001 mbar.

Das Lyophilisat wird mit isotoner Glucoselösung ad 250 ml oder mit Aqua p.i. ad 50 ml rekonstituiert. Der Gehalt beträgt 100 % des Sollgehalts.

| | Liposomeneigenschaften: | |
|---|---|---|
| | vor Lyophilisation | nach Lyophilisation |
| mittlere Größe | 39 nm | 58 nm |
| Dispersitätsindex K2 | 0,530 | 0,630 |
| Trübung | 130 Einheiten | 40 Einheiten |
| Osmolarität | 700 mOsmol | 355 mOsmol |
| pH | 6,5 | 6,1 |

### Beispiel 4

800 g Wasser für Injektionszwecke werden 10 min mit Stickstoff begast. Anschließend werden in dieser Wassermenge 1 g Ascorbinsäure, 50 g Glucose und 1 g L-Arginin gelöst. In diesem Medium werden 1,667 g Nimodipin und 25 g Ei-Phospholipid (z.B. Lipoid E 80) dispergiert und mit stickstoffbegastem Wasser auf 1055,5 g aufgefüllt (Wirkstoff:Phospholipid-Verhältnis 1:15).

Diese Dispersion wird 30 min bei 75°C und unter Stickstoffschutz mit einem schnellaufenden Rührwerk (z.B. Ultra-Turrax) vorhomogenisiert. Die Vordispersion wird durch einen 5 µm-Filter filtriert.

Anschließend wird die Vordispersion bei 75°C, 800 bar und Stickstoffschutz in einem abriebfrei arbeitenden Hochdruckdüsenhomogenisator eine Stunde homogenisiert. Nach dem Abkühlen auf Raumtemperatur erfolgt die Entkeimungsfiltration (0,2 µm). Der Wirkstoffgehalt beträgt bei diesem Wirkstoff:Phospholipid-Verhältnis 1:15) 72% vom Soll.

Die Dispersion wird zu 15,3 ml in 250 ml- oder 50 ml-Flaschen abgefüllt und mit Hilfe des spin- oder shell freezing bei -65°C (Trockeneis-Ethanol-Kühlmischung) zu einem dünnen Produktkuchen eingefroren.

Das eingefrorene Produkt wird auf die auf -50°C vorgekühlten Stellflächen eines Lyophilisators gebracht und bei -30°C und 0,05 mbar 14 h lang getrocknet. Die Nachtrocknung dauert 7 h bei 30°C und 0,001 mbar.

Das Lyophilisat wird mit isotoner Glucoselösung ad 250 ml oder mit Aqua p.i. ad 50 ml rekonstuiert. Der Gehalt der rekonstituierten Dispersion beträgt 77 % des Sollgehalts.

### Beispiel 5

800 g Wasser für Injektionszwecke werden 10 min mit Stickstoff begast. Anschließend werden in dieser Wassermenge 1 g Ascorbinsäure, 23,34 g Glucose und 1 g L-Arginin gelöst. In diesem Medium werden 1,667 g Nimodipin und 11,68 g Ei-Phospholipid (z.B. Lipoid E 80) dispergiert und mit stickstoffbegastem Wasser auf 1055,5 g aufgefüllt (Wirkstoff:Phospholipid-Verhältnis = 1:7).

Diese Dispersion wird 30 min bei 75°C und unter Stickstoffschutz mit einem schnellaufenden Rührwerk (z.B. Ultra-Turrax) vorhomogenisiert. Die Vordispersion wird durch einen 5 µm-Filter filtriert. Auf dem Filter sind Wirkstoffirristalle erkennbar.

Anschließend wird die Vordispersion bei 75°C, 800 bar und Stickstoffschutz in einem abriebfrei arbeitenden Hochdruckdüsenhomogenisator eine Stunde homogenisiert. Nach dem Abkühlen auf Raumtemperatur erfolgt die Entkeimungsfiltration (0,2 µm). Der Gehalt der Liposomen-Dispersion liegt bei 18 % des Sollgehalts.

## Patentansprüche (Patentansprüche für folgende(n) Vertragsstaat(en): AT, BE, CH, DE, DK, FR, GB, IE, IT, LI, LU, MC, NL, PT, SE)

1. Parenteral applizierbare stabile Arzneizubereitung frei von organischen Lösungsmitteln auf Basis von Liposomen mit Phospholipidmembran, wobei das Wirkstoff-Phospholipidverhältnis zwischen 1:20 und 1:100 beträgt und der mittlere Liposomen-Durchmesser zwischen 40 und 200 nm liegt und die nach Rekonstitution einen pH-Wert zwischen 4,5 und 6,5 aufweist.

2. Arzneimittelzubereitung gemäß Anspruch 1, wobei die Liposomenmembran zwischen 20 und 60 Gew.-Teilen Phospholipid enthält und 1 Teil Wirkstoff enthält.

3. Arzneimittelzubereitung gemäß Anspruch 1, die nach Rekonstitution einen osmotischen Druck zwischen 270 und 370 mOsmol aufweist.

4. Arzneimittelzubereitung gemäß Anspruch 1, enthaltend als Wirkstoff ein Dihydropyridin der Formel (I) 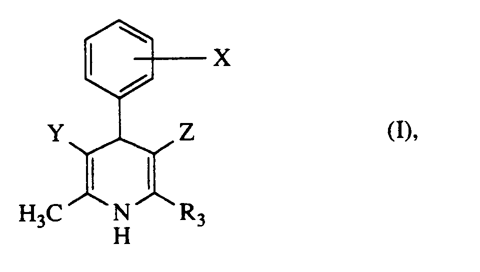 in der
Y die Gruppe NO₂, CN oder -COOR₁ bedeutet, wobei
R₁ für C₁-C₄-Alkyl, gegebenenfalls substituiert durch C₁-C₃-Alkoxy, steht,
Z für die Gruppe CO-NH-Cycloalkyl (3-7 C-Atome) oder -COOR₂ steht, wobei
R₂ C₁-C₁₀-Alkyl, gegebenenfalls substituiert durch C₁-C₃-Alkoxy, Trifluormethyl, N-Methyl-N-benzylamino oder Benzyl bedeutet,
R₃ für C₁-C₄-Alkyl, Cyano oder Hydroxyalkyl (1-4 C-Atome) steht und
X für 1 oder 2 gleiche oder verschiedene Substituenten aus der Gruppe Nitro, Halogen, C₁-C₄-Alkyl, C₁-C₄-Alkoxy, Benzoyl, ein 2,3-Ringglied bestehend aus =N-O-N=, 2-Cycloalkylmethylthio, 2-(4-Methyl-benzyloxy) oder Trifluormethyl steht.

5. Verfahren zur Herstellung von parenteral applizierbarer Arzneizubereitung gemäß Anspruch 1, dadurch gekennzeichnet, daß man 1 Gew.-Teil des Wirkstoffs und 20 bis 60 Gew.-Teile Phospholipid zusammen mit einem Antioxidans, einem Cryoprotector und einem pH-Stabilisator bei Temperaturen zwischen ca. 10°C und 80°C und einem pH zwischen 5 und 7,5 in stickstoffbegastem Wasser mit einem schnellaufenden Rührwerk ausreichend lange vordispergiert.

6. Verfahren gemäß Anspruch 5, dadurch gekennzeichnet, daß man Cryoprotector und Phospholipid in einem Verhältnis zwischen 1:1 und 4:1 einsetzt.

7. Verfahren gemäß Anspruch 5, dadurch gekennzeichnet, daß man als Cryoprotector Glucose einsetzt.

8. Verfahren gemäß Anspruch 5, dadurch gekennzeichnet, daß nach Vordispersion die Liposomen in einem Hochdruckdüsenhomogenisator bei Temperaturen zwischen 20 und 80°C und einem Druck zwischen 400 und 1000 bar auf eine mittlere Größe zwischen 40 und 200 nm homogenisiert werden, und die erhaltene Dispersion gegebenenfalls schnell auf -50°C bis -70°C eingefroren wird.

9. Verfahren gemäß Anspruch 8, dadurch gekennzeichnet, daß nach Homogenisation durch Filtration durch einen 0,2 µm-Filter entkeimt wird.

## Patentansprüche (Patentansprüche für folgende(n) Vertragsstaat(en): ES, GR)

1. Verfahren zur Herstellung von parenteral applizierbarer Arzneizubereitung frei von organischen Lösungsmitteln auf Basis von Liposomen mit Phospholipidmembran, wobei das Wirkstoff-Phopholipidverhältnis zwischen 1:20 und 1:100 beträgt und der mittlere Liposomen-Durchmesser zwischen 40 und 200 nm liegt und die nach Rekonstitution einen pH-Wert zwischen 4,5 und 6,5 aufweist, dadurch gekennzeichnet, daß man 1 Gew.-Teil des Wirkstoffs und 20 bis 60 Gew.-Teile Phospholipid zusammen mit einem Antioxidans, einem Cryoprotector und einem pH-Stabilisator bei Temperaturen zwischen ca. 10°C und 80°C und einem pH zwischen 5 und 7,5 in stickstofbegastem Wasser mit einem schnellaufenden Rührwerk ausreichend lange vordispergiert.

## Claims (Claims for the following Contracting State(s): AT, BE, CH, DE, DK, FR, GB, IE, IT, LI, LU, MC, NL, PT, SE)

1. Stable pharmaceutical preparation which can be administered parenterally and is free of organic solvents, based on liposomes having a phospholipid membrane, the active compound/phospholipid ratio being between 1:20 and 1:100 and the average liposome diameter being between 40 and 200 nm and which, after reconstitution, has a pH of between 4.5 and 6.5.

2. Pharmaceutical preparation according to Claim 1, the liposome membrane containing between 20 and 60 parts by weight of phospholipid and containing 1 part of active compound.

3. Pharmaceutical preparation according to Claim 1 which after reconstitution has an osmotic pressure between 270 and 370 mOsmol.

4. Pharmaceutical preparation according to Claim 1, containing as active compound a dihydropyridine of the formula (I) 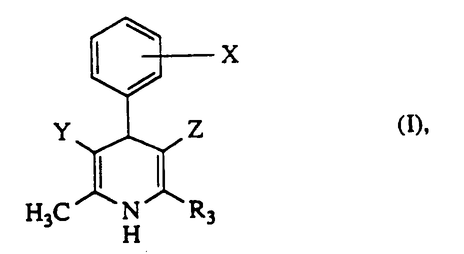 in which
Y denotes the group NO₂, CN or -COOR₁, where
R₁ represents C₁-C₄-alkyl, optionally substituted by C₁-C₃-alkoxy,
Z represents the group CO-NH-cycloalkyl (3-7 C atoms) or -COOR₂, where
R₂ denotes C₁-C₁₀-alkyl, optionally substituted by C₁-C₃-alkoxy, trifluoromethyl, N-methyl-N-benzylamino or benzyl,
R₃ represents C₁-C₄-alkyl, cyano or hydroxyalkyl (1-4 C atoms) and
X represents 1 or 2 identical or different substituents from the group nitro, halogen, C₁-C₄-alkyl, C₁-C₄-alkoxy, benzoyl and a 2,3-ring member consisting of =N-O-N=, 2-cycloalkylmethylthio, 2-(4-methylbenzyloxy) or trifluoromethyl.

5. Process for the production of a pharmaceutical preparation according to Claim 1 which can be administered parenterally, characterised in that 1 part by weight of the active compound and 20 to 60 parts by weight of phospholipid are predispersed for an adequate period together with an antioxidant, a cryoprotector and a pH stabiliser at temperatures between about 10°C and 80°C and a pH between 5 and 7.5 in water into which nitrogen has been passed using a high-speed stirrer.

6. Process according to Claim 5, characterised in that cryoprotector and phospholipid are employed in a ratio of between 1:1 and 4:1.

7. Process according to Claim 5, characterised in that the cryoprotector employed is glucose.

8. Process according to Claim 5, characterised in that after predispersion the liposomes are homogenised to an average size between 40 and 200 nm in a high-pressure jet homogeniser at temperatures between 20 and 80°C and a pressure between 400 and 1000 bar, and the dispersion obtained is optionally rapidly frozen at -50°C to -70°C.

9. Process according to Claim 8, characterised in that after homogenisation the dispersion is sterilised by filtration through a 0.2 µm filter.

## Claims (Claims for the following Contracting State(s): ES, GR)

1. Process for the production of a pharmaceutical preparation which can be administered parenterally and is free of organic solvents, based on liposomes having a phospholipid membrane, the active compound/phospholipid ratio being between 1:20 and 1:100 and the average liposome diameter being between 40 and 200 nm and which, after reconstitution, has a pH of between 4.5 and 6.5, characterised in that 1 part by weight of the active compound and 20 to 60 parts by weight of phospholipid are predispersed for an adequate period together with an antioxidant, a cryoprotector and a pH stabiliser at temperatures between about 10°C and 80°C and a pH between 5 and 7.5 in water into which nitrogen has been passed using a high-speed stirrer.

## Revendications (Revendications pour l'(les) Etat(s) contractant(s) suivant(s): AT, BE, CH, DE, DK, FR, GB, IE, IT, LI, LU, MC, NL, PT, SE)

1. Préparation médicamenteuse stable administrable par voie parentérale, exempte de solvants organiques, à base de liposomes avec membrane phospholipidique dans laquelle le rapport du principe actif et du phospholipide est compris entre 1:20 et 1:100, le diamètre moyen des liposomes est compris entre 40 et 200 nm, et présentant après reconstitution un pH compris entre 4,5 et 6,5.

2. Préparation médicamenteuse selon la revendication 1, dans laquelle la membrane liposomale contient entre 20 et 60 parties en poids de phospholipide et contient 1 partie de principe actif.

3. Préparation médicamenteuse selon la revendication 1, présentant après reconstitution une pression osmotique comprise entre 270 et 370 mOsmol.

4. Préparation médicamenteuse selon la revendication 1, contenant comme principe actif une dihydropyridine de la formule suivante : 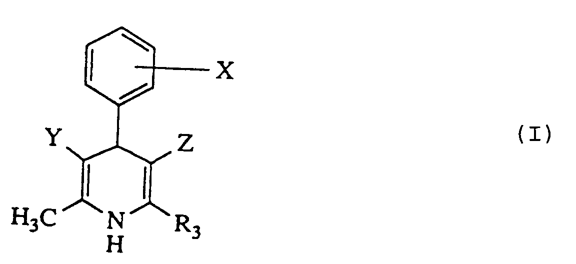 dans laquelle
Y indique le groupe NO₂, CN ou -COOR₁ dans lequel
R₁ représente un radical alkyle en C₁-C₄, le cas échéant substitué par un radical alkoxy en C₁-C₃,
Z indique le groupe CO-NH cycloalkyle (3 à 7 atomes de carbone) ou le groupe -COOR₂, dans lequel
R₂ représente un radical alkyle en C₁-C₁₀, le cas échéant substitué par un radical alkoxy en C₁-C₃, trifluorométhyle, N-méthyl-N-benzylamino ou benzyle,
R₃ indique un groupe alkyle en C₁-C₄, cyano ou hydroxyalkyle (1 à 4 atomes de carbone), et
X indique 1 ou 2 substituants identiques ou différents du groupe nitro, halogène, alkyle en C₁-C₄, alkoxy en C₁-C₄, benzoyle, un élément d'un cycle 2,3 composé de =N-O-N=, 2-cycloalkylméthylthio, 2-(4-méthyl benzyloxy) ou trifluorométhyle.

5. Procédé de production d'une préparation médicamenteuse administrable par voie parentérale selon la revendication 1, caractérisé en ce que l'on disperse au préalable 1 partie en poids du principe actif et 20 à 60 parties en poids de phospholipide en incorporant un antioxydant, un cryoprotecteur et un stabilisateur de pH à des températures comprises entre 10°C et 80°C et à un pH compris entre 5 et 7,5 dans de l'eau sous barbotage à l'azote au moyen d'un agitateur à grande vitesse pendant une durée suffisamment longue.

6. Procédé selon la revendication 5, caractérisé en ce que le cryoprotecteur et le phospholipide sont introduits dans un rapport compris entre 1:1 et 4:1.

7. Procédé selon la revendication 5, caractérisé en ce que le cryoprotecteur utilisé est du glucose.

8. Procédé selon la revendication 5, caractérisé en ce que, après dispersion préalable, les liposomes sont homogénéisés à une taille moyenne comprise entre 40 et 200 nm dans un homogénéisateur à buses haute pression à des températures comprises entre 20°C et 80°C et sous une pression comprise entre 400 et 1000 bars, et en ce que la dispersion obtenue est le cas échéant congelée rapidement à une température de -50°C à -70°C.

9. Procédé selon la revendication 8, caractérisé en ce qu'une stérilisation est effectuée après homogénéisation par filtration sur un filtre de 0,2 µm.

## Revendications (Revendications pour l'(les) Etat(s) contractant(s) suivant(s): ES, GR)

1. Procédé de production d'une préparation médicamenteuse administrable par voie parentérale, exempte de solvants organiques, à base de liposomes avec membrane phospholipidique dans laquelle le rapport du principe actif et du phospholipide est compris entre 1:20 et 1:100, le diamètre moyen des liposomes est compris entre 40 et 200 nm et présente après reconstitution un pH compris entre 4,5 et 6,5, caractérisé en ce que l'on disperse au préalable 1 partie en poids du principe actif et 20 à 60 parties en poids de phospholipide en incorporant un antioxydant, un cryoprotecteur et un stabilisateur de pH à des températures comprises entre 10°C et 80°C et à un pH compris entre 5 et 7,5 dans de l'eau sous barbotage à l'azote au moyen d'un agitateur à grande vitesse pendant une durée suffisamment longue.
